# EUROPEAN PATENT SPECIFICATION

(11) **EP 3 317 261 B1**
(45) Date of publication and mention of the grant of the patent: **24.06.2020**
(21) Application number: 16732637.0
(22) Date of filing: 28.06.2016
(51) Int. Cl.: C07D 307/08

(54) **PROCESS FOR THE PRODUCTION OF 1,4-BUTANEDIOL AND TETRAHYDROFURAN FROM FURAN**
VERFAHREN ZUR HERSTELLUNG VON 1,4-BUTANDIOL UND TETRAHYDROFURAN AUS FURAN
PROCÉDÉ POUR LA PRODUCTION DE 1,4-BUTANEDIOL ET TÉTRAHYDROFURANE À PARTIR DE FURANE

(30) Priority: 30.06.2015 EP 15174599
(43) Date of publication of application: 09.05.2018
(73) Proprietor: Shell Internationale Research Maatschappij B.V., 2596 HR The Hague (NL)
(72) Inventor: LANGE, Jean, Paul, Andre, Marie, Joseph, Ghislain, 1031 HW Amsterdam (NL); HAAN, Rene, Johan, 1031 HW Amsterdam (NL)
(74) Representative: Shell Legal Services IP
(86) International application number: PCT/EP2016/064956
(87) International publication number: WO 2017/001376

(56) References cited:
- BIN ZHANG ET AL: "Selective conversion of furfuryl alcohol to 1,2-pentanediol over a Ru/MnOx catalyst in aqueous phase", GREEN CHEMISTRY, vol. 14, no. 12, 1 January 2012 (2012-01-01), page 3402, XP055122248, ISSN: 1463-9262, DOI: 10.1039/c2gc36270h

## Description

### Field of the Invention

The present invention relates to a process for the production of 1,4-butanediol and THF from furan.

### Background of the Invention

Furan and its derivatives are useful precursors for industrial chemicals. In particular, furan may be converted into 1,4-butanediol (1,4-BDO) and tetrahydrofuran (THF), both of which are valuable industrially-used chemicals.

1,4-BDO is used as an industrial solvent and in the manufacture of polyurethanes and polymers such as polyesters.

THF, on the other hand, is used in the production of elastic fibres such as elastane/spandex (marketed as e.g. Lycra®), as well as being used as an industrial solvent for PVC and in varnishes.

THF and 1,4-BDO are usually produced industrially from petrochemical (fossil fuel) derived feedstocks such as acetylene and propylene, via a number of routes.

An industrial route for the production of 1,4-BDO requires the reaction of acetylene with two equivalents of formaldehyde followed by hydrogenation of the resultant 1,4-butynediol to form 1,4-butanediol. In an alternative process, propylene oxide is converted to allyl alcohol. The allyl alcohol is then hydroformylated to form 4-hydroxybutyraldehyde, which may be hydrogenated to form 1,4-butanediol.

A route that is becoming more popular for the production of 1,4-BDO is the oxidation of butane to maleic anhydride followed by its selective hydrogenation to 1,4-BDO and THF. Other traditional routes for the production of 1,4-BDO use butadiene, allyl acetate or succinic acid as starting materials.

One industrial route for the production of THF proceeds via the hydrogenation of furan over a nickel catalyst (McKillip, W.J., ACS Symposium Series, Issue 385, 1989, Pages 408-423). In turn, Furan may be produced by Paal-Knorr synthesis which reacts 1,4-diketones with phosphorus pentoxide (P₂O₅), or by Feist-Benary synthesis which uses α-halogen ketones and β-dicarbonyl compounds as feedstock. In any event, such feedstocks are fossil fuel derived.

In recent years, to reduce the demand for fossil fuels, increased efforts have focused on producing chemicals, including 1,4-BDO and THF, from non-fossil fuel/renewable feedstocks such as hemicellulose. This route initially involves the production of furfural, followed by its conversion to its derivatives, such as furan.

A method for obtaining furan from renewable resources involves the decarbonylation of furfural. Examples of reaction processes for achieving this, and the subsequent conversion of the furan into its derivatives, can be found in: (i) Hoydonck, H.E., Van Rhijn, W.M., Van Rhijn, W., De Vos, D.E. & Jacobs, P.A. (2012) "Furfural and Derivatives", in Ulmann's Encyclopedia of Industrial Chemistry (volume 16, pp 285-313), Wiley-VCH Verlag GmbH & Co. KGaA; (ii) Dunlop, A.P. and Peters, F.N., in "The Furans" Reinhold Publ. Co, 1953; (iii) K.J. Zeitsch, in "The Chemistry and Technology of Furfural and its Many By-products" Sugar Series 13, Elsevier, 2000; (iv) Lange, J-P, van der Heide, E, van Buijtenen, J., and Price, R. "Furfural-A Promising Platform for Lignocellulosic Biofuels", ChemSusChem 2012, 5, 150 - 166; and (v) Watson, J.M., Ind. Eng. Chem. Prod. Res. Develop., 1973, 12(4), 310.

Furfural may be obtained from hemicellulose via acid hydrolysis in the liquid phase as well as in the gas phase as described in WO 2002/22593 and WO 2012/041990.

The conversion of furan to THF and 1,4-BDO by hydrogenation in the presence of water, acetic acid and Raney nickel or oxide supported nickel catalyst is described in Watson, J.M., Ind. Eng. Chem. Prod. Res. Develop., 1973, 12(4), 310.

A process for the conversion of furan into THF, 1,4-BDO and n-butanol is taught in US 5905159. This document teaches a process in which furan is converted as a reaction mixture with water and in the presence of hydrogen, but in the absence of a water-soluble acid, in a single stage over a hydrogenation catalyst. The hydrogenation catalyst of US 5905159 contains at least one element of subgroup I, V, VI, VII or VIII in the form of a compound or in elemental form, with the restriction that the catalyst does not contain nickel alone. The catalysts taught in US 5905159 generally contain two metals with most containing rhenium as a promoter. The most preferred catalyst taught in US 5905159 for the process contains rhenium and ruthenium supported on active carbon.

Challenges remain concerning the method of production of 1,4-BDO and THF from furan. It is desirable that non-fossil fuel renewable feedstock(s) is/are used for the production of 1,4-BDO and THF, as a contribution to minimising fossil fuel use.

The non-fossil fuel based production processes of 1,4-BDO and THF from furan requires the use of catalyst compositions that contain expensive and rare metals, so the activity, the product selectivity and the life-span of such catalysts, as well as their cost, are of particular concern to the operators of such processes. Therefore, it is in the interest of such operators to prolong the life-span of these catalysts, to maintain their maximum activity for as long as possible, and to keep their product selectivity within the required commercial and operational margins. However, commercial and operational needs may, for example, require reaction conditions to be altered for periods of time in ways that may be unfavourable to the integrity and activity of such catalysts, thus shortening the catalyst's life-span and/or or adversely affecting its product selectivity. It would therefore be advantageous to have available catalysts that can withstand broader operational conditions.

It would also be advantageous to the producers of 1,4-BDO and THF from non-fossil fuel-derived furan to tailor the respective amounts of 1,4-BDO and THF produced so that they can respond quickly to changes in the demand for these products at any given time. A way to respond quickly to such changes in demand is to have multiple reactor vessels, each charged with a different catalyst of a particular product selectivity, so that production can be switched between the reactor vessels depending on the desired product at that particular time. However, this would be an impractical and expensive option as some reactor vessels would have to remain idle during times when the product ratio they can produce is not needed. A simpler, cheaper and a more practical way of altering product ratio remains to be made available.

### Summary of the Invention

Accordingly, the present invention provides a method for increasing the molar ratio of tetrahydrofuran to 1,4-butanediol in a process for the production of 1,4-butanediol and tetrahydrofuran, said process comprising contacting furan with hydrogen and water in a reactor vessel, at an initial partial pressure of hydrogen and in the presence of a catalytic composition comprising at least one metal on a solid support, wherein the at least one metal is selected from cobalt, nickel, ruthenium, palladium and platinum and wherein the molar ratio of tetrahydrofuran to 1,4-butanediol is increased by increasing the partial pressure of hydrogen in the reactor vessel above the initial partial pressure of hydrogen.

The present invention also provides a method for increasing the molar ratio of 1,4-butanediol to tetrahydrofuran in a process for the production of 1,4-butanediol and tetrahydrofuran, said process comprising contacting furan with hydrogen and water in a reactor vessel, at an initial partial pressure of hydrogen and in the presence of a catalytic composition comprising at least one metal on a solid support, wherein the at least one metal is selected from cobalt, nickel, ruthenium, palladium and platinum, and wherein the molar ratio of 1,4-butanediol to tetrahydrofuran is increased by decreasing the partial pressure of hydrogen in the reactor vessel below the initial partial pressure of hydrogen.

### Detailed Description of the Invention

The present inventors have surprisingly found that in the process of reacting furan with hydrogen and water in the presence of a solid-supported catalytic composition comprising at least one metal selected from cobalt, nickel, ruthenium, palladium and platinum, the molar ratio of THF to 1,4-BDO can be tailored to produce the product that suits market demand for 1,4-BDO and THF at the prevailing time. The present inventors have surprisingly found that in such a process, increasing the partial pressure of hydrogen in the reactor vessel leads to more THF being produced relative to 1,4-BDO, and lowering the partial pressure of hydrogen in the reactor vessel leads to more 1,4-BDO being produced relative to THF.

The present inventors have, therefore, found that the abovementioned surprising effects can be utilised to tailor the molar ratio of the products of the process, i.e. THF and 1,4-BDO, with minimal cost and operational burden.

The catalytic composition used in the process of the present invention contains at least one metal selected from the group consisting of cobalt, nickel, ruthenium, palladium and platinum (Co, Ni, Ru, Pd, Pt respectively) on a solid support, more preferably the at least one metal is selected from the group consisting of Ru, Pd, Pt, and most preferably the at least one metal is Ru. The at least one metal may be present on the catalytic composition in its elemental form or as one or more compounds.

Further to the above-mentioned metal or metals, the catalytic composition used in the present invention may contain an additional metal, for example a promotor metal or metals that will at least in part catalyse different reactions, such as ring-opening. A suitable example of such an additional metal is rhenium (Re). The additional metal of the catalytic composition may be present in its elemental form, or as one or more compounds.

The method of application of the at least one metal and, if present, the additional metal to the support is not critical and may be effected in a wide range of ways. Such metals may be applied to the support using the same or different methods and either sequentially of simultaneously. Suitable methods include, for example, impregnation of the support with solutions or suspensions of the salts, complexes, hydroxides, oxides or other organic or inorganic compounds of the relevant metals, drying and optional calcination. Another possibility for applying such metals to the support is to impregnate the latter with a solution of thermally readily decomposable complexes, for example with carbonyl or hydride complexes of the rhenium and/or palladium, and to heat the carrier thus impregnated to, for example, 150°C to 600°C for thermal decomposition of the absorbed metal compounds. Said metals may furthermore be deposited on the catalyst carrier by vapour deposition or by flame spraying. Another suitable preparation method consists of ionexchange of the support with cationic salts or complexes of the metals followed by drying. Subsequent reduction of the metal compound to the relevant metals or compounds of lower oxidation states by means of a reducing agent may be carried out after any method of deposition.

The total amount of the at least one metal selected from Co, Ni, Ru, Pd and Pt (considered as elements) on the catalytic composition may vary within wide ranges, and may be preferably at least 0.01 wt%, more preferably at least 0.02 wt%, more preferably at least 0.03 wt%, more preferably at least 0.1 wt%, more preferably at least 0.3 wt%, most preferably at least 1.0 wt%. Further, preferably, the total amount of the at least one metal selected from Co, Ni, Ru, Pd and Pt (considered as elements) on the catalytic composition is at most 20 wt%, more preferably at most 10 wt%, most preferably at most 3 wt%.

The total amount of the additional metal (considered as its elemental form), if present, on the catalyst may vary within wide ranges, and may be preferably at least 0.02 wt%, more preferably at least 0.03 wt%, more preferably at least 0.1 wt%, more preferably at least 0.3 wt%, most preferably at least 1.0 wt%. Further, preferably, the total amount of the additional metal (considered as its elemental form), if present, on the catalyst is at most 20 wt%, more preferably at most 10 wt%, most preferably at most 5 wt%.

Preferably, the combined total amount of the abovementioned metals (the at least one metal and the additional metal in the catalytic composition), considered as their elemental form may be at least 0.01 wt%, more preferably at least 0.1 wt%, more preferably at least 0.5 wt%, more preferably at least 1.0 wt%. Further, preferably, the total amount of said metal or metals is at most 20 wt%, more preferably at most 10 wt%, most preferably at most 5 wt%.

The composition of the solid support suitably includes oxides of aluminium, titanium, zirconium, silicon, and combinations thereof. The solid support may be amorphous and/or crystalline. The solid support may also comprise clays such as montmorillonite or zeolites, such as ZSM-5 or ZSM-10 zeolites. In another embodiment, the solid support may be composed of carbon, such as active carbon or carbon fibres. The carbon can be amorphous or graphitic, or a mixture of the two. Mixtures of different supports can, of course, also serve as solid supports for the catalytic compositions to be used in the process of the invention. Preferred solid supports comprise zirconium dioxide, titanium oxides and active carbon. More preferably, the solid support comprises titanium dioxide or active carbon. Most preferably, the solid support comprises active carbon.

In the process of the invention, furan is contacted with hydrogen and water in a reactor vessel in the presence of the catalytic composition. The furan may be contacted with hydrogen and water either in the gas or in the liquid phase. Suitable conditions for the production of a mixture of BDO and THF from furan include co-feeding water as a gas or liquid at a molar ratio of water:furan of at least 0.2:1, preferably at least 1:1, and most preferably at least at 3:1. Suitable conditions for the production of a mixture of BDO and THF from furan include co-feeding water as a gas or liquid at a molar ratio of water:furan of at most 100:1, preferably at most 20:1, and most preferably at most 10:1.

Further suitable conditions for the production of a mixture of BDO and THF from furan include the use of a solvent comprising water and/or oxygenates, preferably the reaction product (THF) or the eventually by-products.

In general, the process for the production of 1,4-BDO and THF from furan can be carried out at a molar ratio of hydrogen:furan of at most at 100:1, preferably at most at 10:1, and most preferably at most at 3:1. In general, the process for the production of 1,4-BDO and THF from furan can be carried out at a molar ratio of hydrogen:furan of at least at 0.2:1, preferably at least at 0.5:1, and most preferably at least at 1:1.

In general, the process for the production of 1,4-BDO and THF from furan can be carried out at a reactor temperature at most at 350°C, preferably at most at 250°C, more preferably at most at 200°C, and most preferably at most at 180°C. In general, the process for the production of 1,4-BDO and THF from furan can be carried out at a reactor temperature of at least 25°C, preferably of at least 75°C, more preferably of at least 125°C, and most preferably of at least 140°C.

In general, the process for the production of 1,4-BDO and THF from furan can be carried out at a total reactor pressure of at most 15MPa, preferably of at most 10MPa, more preferably of at most 8MPa, and most preferably of at most 5MPa. In general, the process for the production of 1,4-BDO and THF from furan can be carried out at a total reactor pressure of at least 0.1MPa, preferably of at least 1MPa, more preferably of at least 2MPa, and most preferably of at least 3MPa.

In general, a total reactor pressure above 15MPa is avoided in the process for the production of 1,4-BDO and THF from furan, as this will require the process to be carried out in specialised equipment designed to withstand high pressures and variations in the reaction pressure.

Typically, the process for the production of 1,4-BDO and THF from furan is carried out at an initial total reactor pressure of at least 0.1MPa, preferably at least 2MPa, more preferably at least 4MPa, even more preferably at least 6MPa, and most preferably at least 6MPa. Typically, the initial total reactor pressure is at most 15MPa, preferably at most 13MPa, more preferably at most 11MPa, even more preferably at most 10MPa, and most preferably at most 8MPa.

Typically, the process for the production of 1,4-BDO and THF from furan is carried out at an initial reactor temperature of at least 25°C, preferably at least 50°C, more preferably at least 100°C, even more preferably at least 150°C, and most preferably at least 170°C. Typically, the initial reactor temperature is at most 350°C, preferably at most 300°C, more preferably at most 250°C, even more preferably at most 200°C, and most preferably at most 175°C.

Typically, the process for the production of 1,4-BDO and THF from furan is carried out at an initial molar ratio of water:furan of at least 0.2:1, preferably at least 1:1, and most preferably at least at 3:1. Typically, the initial molar ratio of water:furan of at most 100:1, preferably at most 20:1, and most preferably at most 10:1.

Typically, the process for the production of 1,4-BDO and THF from furan is carried out at an initial molar ratio of hydrogen:furan of at most at 100:1, preferably at most at 10:1, and most preferably at most at 3:1. Typically, the initial molar ratio of hydrogen:furan of at least at 0.2:1, preferably at least at 0.5:1, and most preferably at least at 1:1.

In the process of the present invention, the initial partial pressure of hydrogen in the reactor vessel at any given time is dependent on both the reactor temperature and the total reactor pressure, could readily be determined by the skilled person, and in any event is always lower than the total reactor pressure. Typically, the partial pressure of hydrogen is 2MPa lower that the total reactor pressure at 140°C, and 40MPa lower than the total reactor pressure at 180°C.

In the process of the present invention, the molar ratio of THF to 1,4-BDO produced may be increased by increasing the partial pressure of hydrogen in the reactor vessel above the initial partial pressure of hydrogen in the reactor vessel. The partial pressure of hydrogen in the reactor vessel may be increased above the initial partial pressure of hydrogen in the reactor vessel by a number of methods including, but not limited to, increasing the total reactor pressure above the initial reactor pressure, decreasing the reactor temperature below the initial reactor temperature, increasing the molar ratio of water:furan in the reactor vessel above the initial molar ratio of water:furan, and/or by increasing the molar ratio of hydrogen:furan in the reactor vessel above the initial molar ratio of hydrogen:furan.

In one embodiment of the invention, the partial pressure of hydrogen in the reactor vessel is increased by increasing the total reactor pressure. This may be achieved by, amongst other ways, restricting the exit of gaseous effluent from the reactor vessel, or any other means available in the art to the skilled person. In this embodiment, the total reactor pressure is increased above the initial total reactor pressure preferably by at least 0.5MPa, more preferably by at least 2MPa, even more preferably by at least 5MPa, and most preferably by at least 9MPa. In this embodiment, the total reactor pressure may be increased above the initial reactor pressure in the reactor vessel preferably by at most 13MPa, more preferably by at most 10MPa. The operator of the process of the present invention may select and deploy any such increase in the total reactor pressure above the initial reactor pressure once, or more than once, subject to the total reactor pressure remaining at all times at, or below, 15MPa.

In one embodiment of the invention, the partial pressure of hydrogen in the reactor vessel is increased above the initial partial pressure of hydrogen in the reactor vessel by decreasing the reactor temperature below the initial reactor temperature. In this embodiment, the reactor temperature is decreased below the initial reactor temperature preferably by at least 10°C, more preferably by at least 25°C, even more preferably by at least 50°C, and most preferably by at least 100°C. In this embodiment, the reactor temperature is decreased below the initial the reactor temperature preferably by at most 175°C, more preferably at most by 125°C, even more preferably at most by 50°C, and most preferably at most by 10°C. The operator of the process of the present invention may select and deploy any such decrease in the reactor temperature below the initial reactor temperature once, or more than once, or in any combination, subject to the reactor temperature remaining at all times at, or above, 25°C.

In one embodiment of the invention, the partial pressure of hydrogen in the reactor vessel is increased above the initial partial pressure of hydrogen in the reactor vessel by increasing the molar ratio of water:furan in the reactor vessel. In this embodiment, the molar ratio of water:furan in the reactor vessel is preferably increased above the initial molar ratio of water:furan by a factor of 1.5, more preferably by a factor of 2, even more preferably by a factor of 4, and most preferably by a factor of 10.

In one embodiment of the invention, to increase the molar ratio of THF to 1,4-BDO produced by the process of the present invention, the partial pressure of hydrogen in the reactor vessel is increased above the initial partial pressure of hydrogen in the reactor vessel by increasing the molar ratio of hydrogen:furan in the reactor vessel. In this embodiment, the molar ratio of hydrogen:furan in the reactor vessel is preferably increased above the initial molar ratio of hydrogen:furan by a factor of 1.5, more preferably by a factor of 2, even more preferably by a factor of 4, and most preferably by a factor of 10.

Conversely, in the process of the present invention the molar ratio of 1,4-BDO to THF produced may be increased by lowering the partial pressure of hydrogen in the reactor vessel below the initial partial pressure of hydrogen in the reactor vessel. The partial pressure of hydrogen in the reactor vessel may be decreased below the initial partial pressure of hydrogen in the reactor vessel by a number of methods, including, but not limited to decreasing the total reactor pressure below the initial reactor pressure, increasing the reactor temperature above the initial reactor temperature, decreasing the molar ratio of water:furan in the reactor vessel below the initial molar ratio of water:furan, and/or by decreasing the molar ratio of hydrogen:furan in the reactor vessel below the initial molar ratio of hydrogen:furan.

In one embodiment of the invention, the partial pressure of hydrogen in the reactor vessel is decreased by decreasing the total reactor pressure. This may be achieved by, amongst other ways, facilitating the exit of gaseous effluent, or any other means available in the art to the skilled person. In this embodiment, the total reactor pressure is preferably decreased below the initial total reactor pressure by at least 0.5MPa, preferably by at least 2MPa, more preferably by at least 5MPa, and even more preferably by at least 9MPa. In this embodiment, to decrease the partial pressure of hydrogen in the reactor vessel, the total reactor pressure may be preferably decreased below the initial reactor pressure in the reactor vessel by at most 13MPa, and more preferably by at most 10MPa.

In one embodiment of the invention, the partial pressure of hydrogen in the reactor vessel is decreased below the initial partial pressure of hydrogen by increasing the reactor temperature above the initial reactor temperature. In this embodiment, the reactor temperature is preferably increased above the initial the reactor temperature by at least 10°C, more preferably by at least 25°C, even more preferably by at least 50°C, and most preferably by at least 100°C. In such embodiment, the reactor temperature is increased above the initial the reactor temperature preferably by at most 125°C. The operator of the process of the present invention may select and deploy any such increase in the reactor temperature above the initial reactor temperature once, or more than once, or in any combination, subject to the reactor temperature remaining at all times at, or below, 350°C.

In one embodiment of the invention, the partial pressure of hydrogen in the reactor vessel is decreased below the initial partial pressure of hydrogen by decreasing the molar ratio of water:furan in the reactor vessel. In this embodiment, the molar ratio of water:furan in the reactor vessel is preferably decreased below the initial molar ratio of water:furan by 25%, more preferably by 50%, even more preferably by 75%, most preferably by 90%.

In one embodiment of the invention, to increase the molar ratio of 1,4-BDO to THF produced by the process of the present invention, the partial pressure of hydrogen in the reactor vessel is decreased above the initial partial pressure of hydrogen by decreasing the molar ratio of hydrogen:furan in the reactor vessel. In this embodiment, the molar ratio of hydrogen:furan in the reactor vessel is preferably decreased below the initial molar ratio of hydrogen:furan by 25%, more preferably by 50%, even more preferably by 75%, and most preferably by 90%.

Any suitable reactor vessel may be used for the production of 1,4-BDO and THF from furan. These include, but are not limited to fixed bed and slurry reactors.

The invention will now be illustrated by the following non-limiting examples.

### Example 1

Catalysts were evaluated in a four-barrel microflow unit that consists of 4 parallel Hastelloy HC 276 reactor (1 cm ID). The reactors had an isothermal zone of 25 cm length and an internal volume of 41 mL. The reactors can be operated between 40 and 500°C under 0.15 to 14 MPa pressure. The liquid feed was fed to the reactor by 1000 mL ISCO 1000D pumps with a maximum flow rate of 100 mL/h. Hydrogen was applied to the reactor through a mass flow controller with a maximum flow rate of 5 NL/h.

The catalysts were loaded as crushed (30-80 mesh) particles, as 3 g load, and diluted in an equal weight of SiC (0.2 mm). The catalysts used in each reactor comprised an active carbon support (RX3, commercially available from Norrit) that was impregnated with 4 wt% of Re and 0.04 wt% of Pd.

The initial catalyst reduction was carried at 275°C for 16 h under atmospheric pressure and 1 Nl/h flow of 50 vol% H₂ in N₂ and, subsequently, for 2 h at 4 bara and a 1 NL/h flow of pure 100 % H₂. After reduction the temperature was lowered to 200°C, the hydrogen flow and pressure were set to target and the furan-containing liquid flow was admitted to the reactor.

The reaction was then carried out over a wide operation window for some 900 h time on stream, which is abbreviated herein as TOS. The window covered temperatures of 130-200°C, pressures of 30-130bar, WHSV of 0.2-2/h, and feed concentrations of 12-30w% for furan and 27-34 w% for water with EtOH as balance.

Table 1 reports the ratio of THF to 1,4-BDO produced under the conditions identified therein.

**Table 1**

| (140-180C, 30-110 bar, furan/water/EtOH: 23/30/47 w/w, WHSV=0.46/h, H₂/Furan=2.5) | | | | |
|---|---|---|---|---|
| TOS | P | T | conversion | THF/BDO |
| [h] | [barg] | [C] | [%C] | |
| **48** | **130** | **150** | **100.0** | **14.5** |
| **120** | **100** | **150** | **100.0** | **8.1** |
| **216** | **70** | **150** | **95.6** | **2.1** |
| **288** | **50** | **150** | **58.8** | **1.3** |
| **378** | **70** | **150** | **77.1** | **1.4** |
| | | | | |
| **72** | **130** | **130** | **100.0** | **14.2** |
| **96** | **100** | **130** | **97.5** | **10.8** |
| **240** | **70** | **130** | **56.1** | **2.6** |
| **264** | **50** | **130** | **38.2** | **2.0** |
| **456** | **70** | **130** | **88.1** | **2.1** |

### Example 2

Example 1 was repeated with a catalyst comprising an active carbon support (RX3, commercially available from Norrit) that was impregnated with 5 wt% of Re and 1wt% of Ru. The results are shown in Table 2.

**Table 2**

| (140-180C, 30-110 bar, furan/water/EtOH: 23/30/47 w/w, WHSV=0.46/h, H₂/Furan=2.5) | | | | |
|---|---|---|---|---|
| TOS | P | T | conversion | THF/BDO |
| [h] | [barg] | [C] | [%C] | |
| 24 | **50** | 160 | 88.1 | 1.5 |
| 48 | **70** | 160 | 99.8 | 2.7 |
| 72 | **90** | 160 | 100.0 | 3.9 |
| 90 | **110** | 160 | 100.0 | 6.3 |
| 108 | **50** | 160 | 75.2 | 1.7 |
| 150 | **30** | **160** | 40.9 | 1.0 |
| 204 | 30 | **170** | 37.9 | 0.9 |
| 252 | 30 | **140** | 38.5 | 1.3 |

### Example 3

Example 1 was repeated with a catalyst comprising a TiO₂ support that was impregnated with 10 wt% of Re and 1wt% of Ru. The results are shown in Table 3.

**Table 3**

| (140-180C, 30-110 bar, furan/water/EtOH: 23/30/47 w/w, WHSV=0.46/h, H₂/Furan=2.5) | | | | |
|---|---|---|---|---|
| TOS | P | T | conversion | THF/BDO |
| [h] | [barg] | [C] | [%C] | Mol/mol |
| 24 | **50** | 160 | 99.7 | 6.8 |
| 48 | **70** | 160 | 100.0 | 11.9 |
| 72 | **90** | 160 | 100.0 | 25.2 |
| 90 | **110** | 160 | 100.0 | 46.3 |
| 108 | **50** | 160 | 100.0 | 6.0 |
| 150 | 30 | **160** | 73.0 | 2.4 |
| 204 | 30 | **170** | 59.5 | 1.8 |
| 228 | 30 | **180** | 54.4 | 1.4 |
| 246 | 30 | **140** | 71.1 | 8.3 |

### Example 4

Example 1 was repeated with a catalyst comprising a TiO₂ support that was impregnated with 2.6 wt% of Ru. The results are shown in Table 4.

**Table 4**

| (140-180C, 30-110 bar, furan/water/EtOH: 23/30/47 w/w, WHSV=0.46/h, H₂/Furan=2.5) | | | | |
|---|---|---|---|---|
| TOS | P | T | conversion | THF/BDO |
| [h] | [barg] | [C] | [%C] | Mol/mol |
| 24 | **50** | 160 | 100.0 | 3.9 |
| 48 | **70** | 160 | 100.0 | 6.0 |
| 72 | **90** | 160 | 100.0 | 8.1 |
| 90 | **110** | 160 | 100.0 | 11.1 |
| 108 | **50** | 160 | 100.0 | 3.7 |
| 150 | **30** | **160** | 63.7 | 1.9 |
| 204 | 30 | **170** | 54.2 | 1.5 |
| 228 | 30 | **180** | 39.2 | 1.3 |
| 246 | 30 | **140** | 81.5 | 3.7 |

## Claims

1. A method for increasing the molar ratio of tetrahydrofuran to 1,4-butanediol in a process for the production of 1,4-butanediol and tetrahydrofuran, said process comprising contacting furan with hydrogen and water in a reactor vessel at an initial partial pressure of hydrogen, and in the presence of a catalytic composition comprising at least one metal on a solid support,
wherein the at least one metal is selected from cobalt, nickel, ruthenium, palladium and platinum and
wherein the molar ratio of tetrahydrofuran to 1,4-butanediol is increased by increasing the partial pressure of hydrogen in the reactor vessel above the initial partial pressure of hydrogen.

2. A process according to claim 1, wherein the partial pressure of hydrogen is increased by increasing the total reactor pressure in the reactor vessel by in the range of from 0.5MPa to 10MPa above the initial total reactor pressure.

3. A process according to claim 1, wherein the partial pressure of hydrogen is increased by lowering the reactor temperature in the reactor vessel by in the range of from 10°C to 125°C below the initial reactor temperature.

4. A process according to claim 1, wherein the partial pressure of hydrogen is increased by increasing the molar ratio of water:furan in the reactor vessel by a factor of from 1.5 to 10 times the initial molar ratio of water:furan.

5. A process according to claim 1, wherein the partial pressure of hydrogen is increased by increasing the molar ratio of hydrogen:furan in the reactor vessel by a factor of from 1.5 to 10 times the initial molar ratio of hydrogen:furan.

6. A method for increasing the molar ratio of 1,4-butanediol to tetrahydrofuran in a process for the production of 1,4-butanediol and tetrahydrofuran, said process comprising contacting furan with hydrogen and water in a reactor vessel, at an initial partial pressure of hydrogen and in the presence of a catalytic composition comprising at least one metal on a solid support,
wherein the at least one metal is selected from cobalt, nickel, ruthenium, palladium and platinum, and
wherein the molar ratio of 1,4-butanediol to tetrahydrofuran is increased by decreasing the partial pressure of hydrogen in the reactor vessel below an initial partial pressure of hydrogen.

7. A process according to claim 6, wherein the partial pressure of hydrogen is decreased by decreasing the total reactor pressure in the reactor vessel in the range of from 0.5MPa to 10MPa below the initial total reactor pressure.

8. A process according to claim 6, wherein the partial pressure of hydrogen is decreased by increasing the reactor temperature in the reactor vessel by in the range from 10°C to 125°C above the initial reactor temperature.

9. A process according to claim 6, wherein the partial pressure of hydrogen is decreased by decreasing the molar ratio of water:furan in the reactor vessel from 25% to 90% of the initial molar ratio of water:furan.

10. A process according to claim 6, wherein the partial pressure of hydrogen is decreased by decreasing the molar ratio of hydrogen:furan in the reactor vessel to from 25% to 90% of the initial molar ratio of hydrogen:furan.

11. A process according to claims 1 to 10, wherein the solid support comprises one or more oxides of aluminium, titanium, zirconium or silicon, amorphous or crystalline.

12. A process according to claims 1 to 11, wherein the solid support comprises carbon, as active carbon or carbon fibres, in amorphous or graphitic form, or as a mixture of the amorphous and the graphitic forms.

13. A process according to claims 1 to 12, wherein the catalytic composition comprises rhenium as an additional metal.

14. A process according to any one of claims 1 to 13, wherein the furan is contacted with hydrogen and water in the liquid phase at an initial total reactor pressure of from 0.3MPa to 15MPa, an initial reactor temperature in the range of from 25°C to 300°C, an initial water:furan molar ratio in the range of from 0.2:1 to 100:1, and an initial hydrogen:furan molar ratio in the range of from 0.2:1 to 100:1.

15. A process according to any one of claims 1 to 14, wherein the furan is contacted with hydrogen and water is co-fed at an initial water:furan molar ratio in the range of from 0.2:1 to 100:1, at an initial reactor temperature in the range of from 100°C to 350°C, an initial total reactor pressure of from 0.3MPa to 15MPa and an initial hydrogen:furan molar ratio in the range of from 0.2:1 to 100:1.

## Patentansprüche

1. Verfahren zum Erhöhen des Molverhältnisses von Tetrahydrofuran zu 1,4-Butandiol in einem Verfahren zum Herstellen von 1,4-Butandiol und Tetrahydrofuran, wobei das Verfahren das Inkontaktbringen von Furan mit Wasserstoff und Wasser in einem Reaktorbehälter bei einem anfänglichen Wasserstoffpartialdruck und in Gegenwart einer katalytischen Zusammensetzung, die mindestens ein Metall auf einem festen Träger aufweist, umfasst, wobei das mindestens eine Metall ausgewählt ist aus Kobalt, Nickel, Ruthenium, Palladium und Platin und wobei das Molverhältnis von Tetrahydrofuran zu 1,4-Butandiol erhöht wird, indem der Wasserstoffpartialdruck im Reaktorbehälter über den anfänglichen Wasserstoffpartialdruck erhöht wird.

2. Verfahren nach Anspruch 1, wobei der Wasserstoffpartialdruck erhöht wird, indem der Gesamtreaktordruck im Reaktorbehälter um einen Wert im Bereich von 0,5 MPa bis 10 MPa über den anfänglichen Gesamtreaktordruck erhöht wird.

3. Verfahren nach Anspruch 1, wobei der Wasserstoffpartialdruck durch Absenken der Reaktortemperatur im Reaktorbehälter um einen Wert im Bereich von 10 °C bis 125 °C unter die anfängliche Reaktortemperatur erhöht wird.

4. Verfahren nach Anspruch 1, wobei der Wasserstoffpartialdruck erhöht wird, indem das Molverhältnis von Wasser:Furan im Reaktorbehälter um einen Faktor vom 1,5- bis zum 10fachen des anfänglichen Molverhältnisses von Wasser:Furan erhöht wird.

5. Verfahren nach Anspruch 1, wobei der Wasserstoffpartialdruck erhöht wird, indem das Molverhältnis von Wasserstoff:Furan im Reaktorbehälter um einen Faktor vom 1,5- bis zum 10fachen des anfänglichen Molverhältnisses von Wasserstoff:Furan erhöht wird.

6. Verfahren zum Erhöhen des Molverhältnisses von 1,4-Butandiol zu Tetrahydrofuran in einem Verfahren zum Herstellen von 1,4-Butandiol und Tetrahydrofuran, wobei das Verfahren das Inkontaktbringen von Furan mit Wasserstoff und Wasser in einem Reaktorbehälter bei einem anfänglichen Wasserstoffpartialdruck und in Gegenwart einer katalytischen Zusammensetzung, die mindestens ein Metall auf einem festen Träger aufweist, umfasst, wobei das mindestens eine Metall ausgewählt ist aus Kobalt, Nickel, Ruthenium, Palladium und Platin, und wobei das Molverhältnis von 1,4-Butandiol zu Tetrahydrofuran erhöht wird, indem der Wasserstoffpartialdruck im Reaktorbehälter unter einen anfänglichen Wasserstoffpartialdruck gesenkt wird.

7. Verfahren nach Anspruch 6, wobei der Wasserstoffpartialdruck durch Absenken des Gesamtreaktordrucks im Reaktorbehälter im Bereich von 0,5 MPa bis 10 MPa unter den anfänglichen Gesamtreaktordruck gesenkt wird.

8. Verfahren nach Anspruch 6, wobei der Wasserstoffpartialdruck durch Erhöhen der Reaktortemperatur im Reaktorbehälter um einen Wert im Bereich von 10 °C bis 125 °C über die anfängliche Reaktortemperatur gesenkt wird.

9. Verfahren nach Anspruch 6, wobei der Wasserstoffpartialdruck durch Verringern des Molverhältnisses von Wasser:Furan im Reaktorbehälter von 25 % auf 90 % des anfänglichen Molverhältnisses von Wasser:Furan verringert wird.

10. Verfahren nach Anspruch 6, wobei der Wasserstoffpartialdruck durch Verringern des Molverhältnisses von Wasserstoff:Furan im Reaktorbehälter auf 25 % bis 90 % des anfänglichen Molverhältnisses von Wasserstoff:Furan verringert wird.

11. Verfahren nach Anspruch 1 bis 10, wobei der feste Träger ein oder mehrere amorphe oder kristalline Oxide von Aluminium, Titan, Zirkonium oder Silizium umfasst.

12. Verfahren nach Anspruch 1 bis 11, wobei der feste Träger Kohlenstoff als Aktivkohle oder Kohlenstofffasern in amorpher oder graphitischer Form oder als Mischung der amorphen und der graphitischen Form umfasst.

13. Verfahren nach Anspruch 1 bis 12, wobei die katalytische Zusammensetzung Rhenium als zusätzliches Metall enthält.

14. Verfahren nach einem der Ansprüche 1 bis 13, wobei das Furan mit Wasserstoff und Wasser in der flüssigen Phase bei einem anfänglichen Gesamtreaktordruck von 0,3 MPa bis 15 MPa, bei einer anfänglichen Reaktortemperatur im Bereich von 25 °C bis 300 °C, bei einem anfänglichen Molverhältnis von Wasser:Furan im Bereich von 0,2:1 bis 100:1 und bei einem anfänglichen Molverhältnis von Wasserstoff:Furan im Bereich von 0,2:1 bis 100:1 in Kontakt gebracht wird.

15. Verfahren nach einem der Ansprüche 1 bis 14, wobei das Furan mit Wasserstoff in Kontakt gebracht wird und Wasser bei einem anfänglichen Molverhältnis von Wasser:Furan im Bereich von 0,2:1 bis 100:1, bei einer anfänglichen Reaktortemperatur im Bereich von 100 °C bis 350 °C, einem anfänglichen Gesamtreaktordruck von 0,3 MPa bis 15 MPa und einem anfänglichen Molverhältnis von Wasserstoff:Furan im Bereich von 0,2:1 bis 100:1 zugeführt wird.

## Revendications

1. Procédé permettant d'augmenter le rapport molaire du tétrahydrofurane au 1,4-butanediol dans un procédé de production de 1,4-butanediol et de tétrahydrofurane, ledit procédé comprenant la mise en contact de furanne avec de l'hydrogène et de l'eau dans une cuve de réacteur à une pression partielle initiale d'hydrogène, et en présence d'une composition catalytique comprenant au moins un métal sur un support solide, dans lequel l'au moins un métal est choisi parmi le cobalt, le nickel, le ruthénium, le palladium et le platine, et dans lequel le rapport molaire du tétrahydrofurane au 1,4-butanediol est augmenté par l'augmentation de la pression partielle d'hydrogène dans la cuve de réacteur au-dessus de la pression partielle initiale d'hydrogène.

2. Procédé selon la revendication 1, dans lequel la pression partielle d'hydrogène est augmentée par l'augmentation de la pression totale du réacteur dans la cuve de réacteur de 0,5 à 10 MPa au-dessus de la pression totale initiale du réacteur.

3. Procédé selon la revendication 1, dans lequel la pression partielle d'hydrogène est augmentée par l'abaissement de la température du réacteur dans la cuve de réacteur de 10 à 125 °C en dessous de la température initiale du réacteur.

4. Procédé selon la revendication 1, dans lequel la pression partielle d'hydrogène est augmentée par l'augmentation du rapport molaire eau:furane dans la cuve de réacteur d'un facteur de 1,5 à 10 fois le rapport molaire eau:furane initial.

5. Procédé selon la revendication 1, dans lequel la pression partielle d'hydrogène est augmentée par l'augmentation du rapport molaire hydrogène:furane dans la cuve de réacteur d'un facteur de 1,5 à 10 fois le rapport molaire hydrogène:furane initial.

6. Procédé permettant d'augmenter le rapport molaire du 1,4-butanediol au tétrahydrofurane dans un procédé de production de 1,4-butanediol et de tétrahydrofurane, ledit procédé comprenant la mise en contact de furanne avec de l'hydrogène et de l'eau dans une cuve de réacteur, à une pression partielle initiale d'hydrogène, et en présence d'une composition catalytique comprenant au moins un métal sur un support solide, dans lequel l'au moins un métal est choisi parmi le cobalt, le nickel, le ruthénium, le palladium et le platine, et dans lequel le rapport molaire du 1,4-butanediol au tétrahydrofurane est augmenté par la diminution de la pression partielle d'hydrogène dans la cuve de réacteur en dessous d'une pression partielle initiale d'hydrogène.

7. Procédé selon la revendication 6, dans lequel la pression partielle d'hydrogène est diminuée par la diminution de la pression totale du réacteur dans la cuve de réacteur de 0,5 à 10 MPa en dessous de la pression totale initiale du réacteur.

8. Procédé selon la revendication 6, dans lequel la pression partielle d'hydrogène est diminuée par l'augmentation de la température du réacteur dans la cuve de réacteur de 10 à 125 °C au-dessus de la température initiale du réacteur.

9. Procédé selon la revendication 6, dans lequel la pression partielle d'hydrogène est diminuée par la diminution du rapport molaire eau:furane dans la cuve de réacteur de 25 % à 90 % du rapport molaire eau:furane initial.

10. Procédé selon la revendication 6, dans lequel la pression partielle d'hydrogène est diminuée par la diminution du rapport molaire hydrogène:furane dans la cuve de réacteur de 25 % à 90 % du rapport molaire hydrogène:furane initial.

11. Procédé selon les revendications 1 à 10, dans lequel le support solide comprend un ou plusieurs oxydes d'aluminium, de titane, de zirconium ou de silicium, amorphes ou cristallins.

12. Procédé selon les revendications 1 à 11, dans lequel le support solide comprend du carbone, sous forme de carbone actif ou de fibres de carbone, sous forme amorphe ou graphitique, ou un mélange des formes amorphe et graphitique.

13. Procédé selon les revendications 1 à 12, dans lequel la composition catalytique comprend du rhénium comme métal supplémentaire.

14. Procédé selon l'une quelconque des revendications 1 à 13, dans lequel le furanne est mis en contact avec de l'hydrogène et de l'eau dans la phase liquide à une pression initiale totale du réacteur de 0,3 à 15 MPa, une température initiale du réacteur comprise entre 25 et 300 °C, un rapport molaire eau:furane initial compris entre 0,2:1 et 100:1 et un rapport molaire hydrogène:furane initial compris entre 0,2:1 et 100:1.

15. Procédé selon l'une quelconque des revendications 1 à 14, dans lequel le furane est mis en contact avec de l'hydrogène et de l'eau est co-alimentée à un rapport molaire eau:furane initial compris entre 0,2:1 et 100:1, à une température initiale du réacteur comprise entre 100 et 350 °C, une pression totale initiale du réacteur de 0,3 à 15 MPa et un rapport molaire hydrogène:furane initial compris entre 0,2:1 et 100:1.
